# EUROPÄISCHE PATENTSCHRIFT

(11) **EP 3 801 070 B1**
(45) Veröffentlichungstag und Bekanntmachung des Hinweises auf die Patenterteilung: **06.09.2023**
(21) Anmeldenummer: 18734752.1
(22) Anmeldetag: 05.06.2018
(51) Int. Cl.: A61Q 19/00, C09K 23/00, C09K 23/14, A23L 5/00, A61K 8/55, A61K 8/06, A61K 31/685, A61K 8/34, A61K 9/107, A61P 3/02, A61K 9/00, A23L 35/00

(54) **ZWEIPHASIGES SYSTEM**
TWO-PHASE SYSTEM
SYSTÈME À DEUX PHASES

(43) Veröffentlichungstag der Anmeldung: 14.04.2021
(73) Patentinhaber: PM-International AG, 5445 Schengen (LU)
(72) Erfinder: SORG, Rolf, 5444 Schengen (LU); WAJDA, Rudi, 68259 Mannheim (DE); MEINHARDT, Horst, 56249 Herschbach (DE); KÜHNE, Tobias, 54497 Morbach (DE); MESSER, Wilhelm, 67098 Bad Dürkheim (DE)
(74) Vertreter: Meissner Bolte Partnerschaft mbB
(86) Internationale Anmeldenummer: PCT/EP2018/064675
(87) Internationale Veröffentlichungsnummer: WO 2019/233552

(56) Entgegenhaltungen:
- EP-A1- 1 927 287
- EP-A1- 2 537 515
- WO-A1-00/37042
- WO-A1-2008/000534
- CN-C- 100 536 826
- DE-A1- 10 255 195
- DE-A1- 19 608 137
- US-A1- 2005 048 088
- US-A1- 2017 304 452
- LIPOID GMBH: "Water soluble composition containing [omega]-3-fatty acids for use in dietetics and food", RESEARCH DISCLOSURE, KENNETH MASON PUBLICATIONS, HAMPSHIRE, UK, GB, Bd. 527, Nr. 13, 1. März 2008 (2008-03-01) , Seite 211, XP007138010, ISSN: 0374-4353
- "Water soluble composition containing [omega]-3-fatty acids for use in dietetics and food", RESEARCH DISCLOSURE, KENNETH MASON PUBLICATIONS, HAMPSHIRE, UK, GB, vol. 527, no. 13, 1 March 2008 (2008-03-01), page 211, XP009534708, ISSN: 0374-4353

## Beschreibung

Die Erfindung betrifft ein zweiphasiges System. Ferner bezieht sich die Erfindung auf ein Verfahren zur Herstellung eines erfindungsgemäßen zweiphasigen Systems. Die Erfindung betrifft auch die Verwendung eines erfindungsgemäßen zweiphasigen Systems als Nahrungsergänzungsmittel oder kosmetisches Präparat.

Die EP 2 537 515 A1 betrifft eine flüssige, wässrige Emulsion sowie deren Verwendung als Getränk und insbesondere als Diätetikum. Darüber hinaus betrifft diese Veröffentlichung eine pharmazeutische Zubereitung, umfassend die flüssig, wässrige Emulsion, insbesondere zur Prophylaxe und Therapie mitochondrialer Dysfunktionen.

Die CN 100 536 826 C beschreibt eine emulsionsartige Formulierung auf Grundlage von Schaffett und Glycerin zur Verwendung auf dem Gebiet der Kosmetik.

Die Veröffentlichung "Water soluble composition containing [omega]-3-fatty acids for use in dietetics and food" (Research Disclosure, Kenneth Mason Publications, Hampshire, UK, GB, Bd. 527, Nr. 13, 1. März 2008, Seite 211, XP007138010, ISSN: 0374-4353) beschreibt wasserlösliche Zusammensetzungen mit Omega-3-Fettsäuren.

Die DE 102 55 195 A1 betrifft das Solubilisieren lipophiler Substanzen, insbesondere aus der Gruppe der Lipide, Steroide, Terpene und polaren Lipide, mittels einer Lecithin/Polyol- bzw. einer Lecithin/Kohlenhydrat-Matrix zu wasserlöslichen und emulsionsartigen Konzentraten zur Verwendung in kosmetischen, diätetischen und pharmazeutischen Artikeln.

Zweiphasige Systeme wie zum Beispiel Emulsionen stellen eine wichtige Darreichungsform dar, sowohl für Nahrungsergänzungsmittel als auch für kosmetische Präparate. Emulsionen sind komplexe zweiphasige Systeme aus zwei nicht miteinander mischbaren Phasen, von denen eine innere Phase in Tropfenform in einer äußeren Phase stabil verteilt vorliegt. Häufig ist die äußere Phase eine wässrige Phase, die innere Phase wird durch ein Öl gebildet. Drei Arten der Stabilität bestimmen die langfristige Qualität und Lagereigenschaften von Emulsionen und ähnlichen zweiphasigen Systemen: Die physikalische Stabilität, die chemische Stabilität und die mikrobiologische Stabilität.

Die physikalische Stabilität beschreibt das Vermögen einer Emulsion bzw. eines zweiphasigen Systems, die bei der Emulgierung durch mechanische Kräfte erreichte, feine und gleichmäßige Verteilung der dispersen Phase über einen längeren Zeitraum beizubehalten. Sie kann beispielsweise durch die Verwendung von Tensiden bzw. Emulgatoren, eine Angleichung der Dichte der beiden Phasen oder eine Erhöhung der Viskosität der äußeren Phase durch sogenannte Quasiemulgatoren, verbessert werden. Die Tenside werden in anionische, kationische, amphotere und nichtionische Tenside unterteilt, wobei die nichtionischen Tenside einige Vorteile aufweisen: Sie bilden im Wässrigen keine Ionen, reagieren neutral, sind nicht durch Elektrolyte beeinflussbar und indifferenter gegenüber chemischen Einflüssen.

Insbesondere im diätetischen und kosmetischen Bereich sind transparente oder zumindest durchscheinende Emulsionen und ähnliche zweiphasige Systeme erwünscht. Dies kann in manchen Fällen durch eine innere Phase mit Tröpfchengrö-βen im Nanometerbereich erreicht werden. Solche Tröpfchengrößen werden beispielsweise durch Micellen, deren Bildung durch einen Emulgator und einen Coemulgator vermittelt werden kann, erreicht. Dabei werden als Emulgator häufig ethoxylierte Tenside bzw. synthetische Tenside mit hohem HLB-Wert und als Coemulgator Alkohole eingesetzt. Um Öle zu emulgieren, ist dabei oft eine erhebliche Menge an Emulgator und Coemulgator erforderlich. Allerdings werden synthetische Emulgatoren von einem Teil der Bevölkerung abgelehnt. Außerdem sind Alkohole wie Ethanol und Propanol Zellgifte und müssen insbesondere bei oralen Zubereitungen für Kinder, Alkoholiker, Schwangere etc. vermieden werden.

Neben der physikalischen Stabilität einer Emulsion oder eines ähnlichen zweiphasigen Systems muss auch die chemische und mikrobiologische Stabilität sichergestellt werden. Die chemische Stabilität ist in zweiphasigen Systemen insbesondere ein Problem, da Autoxidation und hydrolytische Vorgänge in Ölen und Fetten in der Anwesenheit von Wasser begünstigt sind. Ferner werden bei der Herstellung von Emulsionen häufig Luft und damit Sauerstoff eingearbeitet, welcher ebenfalls die Oxidation begünstigt. Dies ist insofern problematisch, da beispielweise oxidierte Fettsäuren nicht mehr nur nicht für physiologische Zwecke zur Verfügung stehen, sondern bei der Oxidation gesundheitsschädliche Produkte entstehen.

Emulsionen und andere zweiphasige Systeme stellen als wasserhaltige Systeme gute Nährböden für Mikroorganismen dar. Daher werden solchen zweiphasigen Systemen üblicherweise Konservierungsmittel beigefügt. Allerdings können Tenside die Wirksamkeit der Konservierungsmittel beeinträchtigen. Da die Konservierungsmittel in der Regel lipophil sind, gehen diese außerdem teilweise in die lipophile Phase über. Damit die Konzentration an Konservierungsmittel in der wässrigen Phase ausreichend hoch ist um eine Konservierung zu bewirken, weisen solche zweiphasigen Systeme einen relativ hohen Gehalt an Konservierungsmitteln auf. Allerdings können bestimmte Konservierungsmittel, beispielsweise die p-Hydroxybenzoesäureester, Unverträglichkeiten und Allergien auslösen.

Viele Menschen wünschen sich Produkte auf Basis verträglicher und natürlich vorkommender Inhalts- bzw. Hilfsstoffe, obgleich dies oft technisch schwer zu erreichen ist. Gerade Emulsionen und ähnliche zweiphasige Systeme stellen komplexe Systeme dar, in welchen Inhaltsstoffe nicht beliebig zusammengemischt werden können. Es muss vielmehr eine Mischung entwickelt werden, die den Bedürfnissen des Anwenders und den hohen technischen Anforderungen an die physikalische, chemische und mikrobiologische Stabilität gerecht wird.

Viele zweiphasige Systeme weisen das Problem auf, dass sie nicht tropffähig sind und sich nicht ohne nennenswerten Verlust an lipophiler Phase durch eine Applikationsvorrichtung dosieren lassen. Verluste treten beispielsweise auf, wenn Teile des zweiphasigen Systems an Applikationsvorrichtungen wie Messbechern haften bleibt. Durch den Verlust sinkt die Menge verabreichter lipophiler Inhaltsstoffe in unvorhersehbarer Weise.

Der vorliegenden Erfindung liegt daher die Aufgabe zu Grunde, ein zweiphasiges System anzugeben, das bekömmlich ist, keine Allergien auslöst und vom Menschen psychologisch und physiologisch positiv aufgenommen wird. Das zweiphasige System soll transparent sein und keine einwertigen Alkohole, insbesondere kein Ethanol oder Propanol enthalten. Ferner soll das zweiphasige System physikalisch, chemisch und mikrobiologisch stabil sein, obwohl möglichst nur natürlich vorkommende Inhalts- bzw. Hilfsstoffe eingesetzt werden. Auf synthetische Emulgatoren und Konservierungsmittel soll verzichtet werden. Das zweiphasige System soll zur leichten Applikation tropffähig sein und sich ohne nennenswerten Verlust an lipophiler Phase durch die Applikationsvorrichtung dosieren lassen. Ferner ist es eine Aufgabe der Erfindung, ein Verfahren zur Herstellung eines erfindungsgemäßen zweiphasigen Systems und die Verwendung eines erfindungsgemäßen zweiphasigen Systems als Nahrungsergänzungsmittel oder kosmetisches Präparat anzugeben.

Erfindungsgemäß wird diese Aufgabe durch ein zweiphasiges System nach Anspruch 1 gelöst. Mit Blick auf das Verfahren zur Herstellung eines erfindungsgemä-βen zweiphasigen Systems wird die Aufgabe durch Anspruch 10 gelöst. Mit Blick auf die Verwendung eines erfindungsgemäßen zweiphasigen Systems wird die Aufgabe durch Anspruch 11 gelöst.

Die Erfindung beruht auf dem Gedanken, ein zweiphasiges System anzugeben, das eine lipophile Phase und eine wässrige Phase umfasst, wobei das zweiphasige System Lecithine und 0,02 Gew.-% bis 7,77 Gew.-%, besonders bevorzugt 0,02 Gew.-% bis 7,52 Gew.-%, Lipid enthält und die Summe an Gew.-% Lecithinen und Lipid an dem zweiphasigen System 0,5 Gew.-% bis 8,25 Gew.-%, besonders bevorzugt 0,5 Gew.-% bis 8,0 Gew.-%, beträgt. Ferner enthält das zweiphasige System 68 Gew.-% bis 91 Gew.-% Glycerin und keine Inhaltsstoffe tierischer Herkunft. Die lipophile Phase bildet Mizellen mit einer mittleren Tröpfchengröße zwischen 10 nm und 250 nm, die wässrige Phase enthält 75 Gew.-% - 99 Gew.-% Glycerin und das zweiphasige System enthält einen Bestandteil ausgewählt aus Algenöl, Sojaöl, Sonnenblumenöl, Borretschöl, Leinöl, Weizenkeimöl, Sägepalmenöl, Roggenextrakt, Ubichinon-10 und Ubiquinol.

In dem zweiphasigen System verbleibt die lipophile Phase über einen langen Zeitraum fein und homogen dispergiert in der wässrigen Phase. Somit ist das erfindungsgemäße zweiphasige System in genannter Zusammensetzung überraschenderweise physikalisch stabil. Dies ist insbesondere überraschend, da als Emulgatoren nur Lecithine eingesetzt werden und diese gegenüber der zu emulgierenden Substanz in geringen Mengen eingesetzt werden können. Beispielsweise kann die Masse an Lecithinen nur 5 % der Masse des zu emulgierenden Lipides betragen. Ein weiterer Vorteil ist, dass Lecithine natürlichen Ursprungs sind.

Einwertige Alkohole wie zum Beispiel Ethanol oder Propanol sind für die Stabilität des zweiphasigen Systems nicht erforderlich. Gleichzeitig ist deren Einsatz in Ausführungsformen des erfindungsgemäßen zweiphasigen Systems aber nicht zwangsläufig ausgeschlossen. Gleiches gilt für synthetische und/oder ethoxylierte Tenside. Ein Vorteil ist, dass das erfindungsgemäße zweiphasige System bei Verzicht auf einwertige Alkohole wie zum Beispiel Ethanol oder Propanol für Kinder, Alkoholiker, Schwangere etc. geeignet ist. Ein weiterer Vorteil ist, dass das erfindungsgemäße zweiphasige System bei Verzicht auf synthetische und/oder ethoxylierte Tenside auch für Personen geeignet ist, die solche Tenside ablehnen.

Überraschend ist auch, dass das Glycerin eine ausreichende Konservierung vermittelt und keine weiteren Konservierungsmittel erforderlich sind. Dadurch kann auf synthetische oder allergieauslösende Konservierungsmittel verzichtet werden. Die konservierende Wirkung des Glycerins erfolgt durch die Reduzierung der Wasseraktivität in der wässrigen Phase. Möglicherweise können an Stelle des Glycerins oder zusätzlich zu dem Glycerin Kohlenhydrate wie Monosaccharide, Disaccharide, Maltit, Maltodextrin, und/oder Polyole mit mehr als zwei Alkoholgruppen wie z.B. Threit, Pentite, Hexite einzeln oder in Kombination eingesetzt werden, wenn diese die Wasseraktivität in der wässrigen Phase in ausreichender Weise reduzieren.

Ferner liegt oxidations- und hydrolyseempfindliches Lipid überraschenderweise trotz Anwesenheit von Wasser und Luft stabil vor. Dass eine Oxidation vermieden wird, erhöht nicht nur die Menge zur Verfügung stehenden Lipides, sondern verhindert auch die Bildung gesundheitsschädlicher Produkte.

Überraschenderweise hat sich gezeigt, dass das zweiphasige System durch den geringen Massenanteil an Lecithinen und Lipid tropffähig und damit leicht aus Flaschen, Beuteln etc. applizierbar ist. Zweiphasige Systeme mit einem solch geringen Massenanteil an Lecithinen und Lipid lassen sich ohne nennenswerten Verlust an lipophiler Phase durch eine Applikationsvorrichtung dosieren, da das zweiphasige System nicht an Messbechern, Flaschen, Beuteln etc. haften bleibt. Dadurch kann eine gleichbleibende Dosis an Lipid verabreicht werden und weniger Produkt geht bei der Anwendung verloren.

Nur mittels natürlich vorkommender Inhaltsstoffe wird also ein stabiles zweiphasiges System gebildet. Dadurch wird das zweiphasige System physiologisch und psychologisch besonders positiv angenommen und ist besonders gut verträglich. Das zweiphasige System weist ein geringes Risiko für Unverträglichkeiten wie Allergien auf. Überraschenderweise hat sich gezeigt, dass das zweiphasige System eine hohe Transparenz aufweist, woraus ein optisch attraktives Produkt resultiert. Die Transparenz resultiert zum einem aus dem geringen Massenanteil an Lecithinen und Lipid. Vor allem aber weisen Glycerin und Triglyceride einen ähnlichen Brechungsindex auf. Indem die wässrige Phase des zweiphasigen Systems auf einen sehr hohen Gehalt an Glycerin eingestellt wurde, konnten die Erfinder den Brechungsindex der beiden Phasen angleichen, wodurch das zweiphasige System überaschenderweise transparent erscheint.

Letztlich erlaubt es das zweiphasige System, gleichzeitig hydrophile und hydrophobe Verbindungen aufzunehmen, um die Eigenschaften des zweiphasigen Systems weiter zu modifizieren oder um das zweiphasige System als Trägersystem für bestimmte Verbindungen zu nutzen.

Der Ausdruck "zweiphasiges System" beschreibt ein mindestens zweiphasiges System, das eine wässrige Phase mit hohem Glyceringehalt und eine lipophile Phase mit hohem Gehalt an Lipid umfasst. Zwischen wässriger und lipophiler Phase finden sich als Emulgator Lecithine. In bestimmten Ausführungsformen kann das System auch mehr als zwei Phasen aufweisen. In bestimmten Ausführungsformen stellt das zweiphasige System eine Emulsion dar.

Das zweiphasige System enthält mindestens einen der nachgenannten Stoffe als Lipid. Mit anderen Worten kann der Begriff Lipid eine Verbindung in Reinform oder ein Gemisch von Verbindungen bezeichnen. Insbesondere gelten als Lipid einzeln oder in Kombinationen: Fettsäuren und deren Ester, insbesondere Triglyceride und Ethylester, inbesondere pflanzlichen, tierischen und/oder marinen Ursprungs; Omega-3-Fettsäuren wie z.B. Docosahexaensäure (DHA) und/oder Eicosapentaensäure (EPA) und deren Veresterungsprodukte; die fettlöslichen Vitamine sowie Carotinoide, wie z.B. Lutein und Zeaxanthin; Vitamin A (Retinol) und dessen Derivate Retinal, Retinylpalmitat und Retinylacetat; Vitamin E als Sammelbegriff für Tocopherole und Tocotrienole (darunter fallen alpha-, beta-, gamma-, delta- Tocopherole und Tocotrienole, sowohl als natürliche Gemische als auch synthetisch in Reinform); Vitamin E-Derivate wie alpha-, beta-, gamma- und delta-Tocopherolacetat sowohl in optisch reiner Form als auch als Racemat; Vitamin D2 (Ergocalciferol), Vitamin D3 (Cholecalciferol), Vitamin K1 (Phyllochinon), Vitamin K2 (Menachinon), Vitamin K3 (Menadion) und Coenzym Q 10 H (Ubichinol) und Ubichinon-10; Perfluorcarbone;
Das Lipid kann im Herstellungsverfahren des zweiphasigen Systems als isolierte Verbindung oder in Form eines Gemisches, z.B. als Bestandteil(e) eines Öles oder Fettes, oder in Kombinationen davon zugegeben werden. Als zugegebene Öle kommen insbesondere pflanzliche Öle wie Sojaöl, Sonnenblumenöl, Borretschöl, Leinöl, Weizenkeimöl, Sägepalmenöl, Roggenextrakt, Hanföl, insbesondere THC-freies Hanföl, und Orangenöl in Frage. Ferner Öle, die reich an Omega-3-Fettsäuren sind, wie zum Beispiel Algenöl.

Im Rahmen dieser Erfindung werden insbesondere entölte Lecithinfraktionen eingesetzt. Dies bedeutet, dass die Lecithine im Wesentlichen frei von unpolaren Lipiden wie Fettsäuren und Triglyceriden sind. Der Gehalt an unpolaren Lipiden wie Fettsäuren und Triglyceriden beträgt bevorzugt weniger als 3 Gew.-%. Die Lecithine enthalten bevorzugt einen Anteil an polaren Lipiden (acetonunlöslicher Substanz) von 90 bis 100 Gew.-%. Die Lecithine umfassen entölte Fraktionen mit einem bevorzugten Gehalt an Phosphatidylcholin von 30 Gew.-% bis 100 Gew.-%.

Bei der Berechnung von Gew.-% werden im Zweifelsfall jene lipophilen Verbindungen, die einen Phosphat-/Phosphorsäurerest aufweisen, als Lecithine und nicht als Lipid angesehen, falls sich aus der Beschreibung nichts Anderes ergibt. Lipophile Substanzen, die keinen Phosphat-/Phosphorsäurerest aufweisen, werden im Zweifelsfall als Lipid angesehen, falls sich aus der Beschreibung nichts Anderes ergibt.

Bevorzugte Ausführungsformen der Erfindung sind in den Unteransprüchen angegeben.

Bevorzugt enthält das zweiphasige System 0,48 Gew.-% bis 8,46 Gew.-%, bevorzugt 0,48 Gew.-% bis 8,21 Gew.-%, besonders bevorzugt 0,48 Gew.-% bis 7,96 Gew.-%, Lecithine.

Bevorzugt enthalten die Lecithine 40 Gew.-% bis 90 Gew.-% Phosphatidylcholin.

Die im Rahmen dieser Erfindung verwendeten Lecithine stammen bevorzugt aus Soja, Sonnenblume, Raps, Fisch, Milch und/oder Eiern, wobei nichttierische Quellen wie Soja, Sonnenblume und/oder Raps bevorzugt sind.

Die wässrige Phase enthält 75 Gew.-% - 99 Gew.-% Glycerin. Ein hoher Gehalt an Glycerin in der wässrigen Phase gewährt eine ausreichende Konservierung des zweiphasigen Systems.

In bevorzugten Ausführungsformen beträgt in dem zweiphasigen System das Massenverhältnis an Lecithinen zu Lipid zwischen 8:1 und 1:20, besonders bevorzugt zwischen 1:1 und 1:12.

In bestimmten bevorzugten Ausführungsformen enthält das zweiphasige System ein Algenöl, insbesondere ein Algenöl aus Ulkenia sp. und/oder Schizochytrium sp.

Algenöl ist ähnlich reich an Omega-3-Fettsäuren wie bestimmte Fischöle und weist den Vorteil auf, dass es bekömmlicher ist und weniger Aufstoßen verursacht. Es weist vor allem nicht den typischen Geruch und Geschmack von Fisch auf. Darüber hinaus ist es für eine vegetarische oder vegane Ernährung geeignet.

Omega-3-Fettsäuren wie beispielsweise die Docosahexaensäure (DHA) und die Eicosapentaensäure (EPA) bilden einen wichtigen Teil der menschlichen Ernährung. Häufig wird der Bedarf an Omega-3-Fettsäuren nicht durch die Ernährungsgewohnheiten bzw. -möglichkeiten erfüllt. Erhältliche Omega-3-Präparate, insbesondere in Form von Kapseln mit Fischöl, werden häufig als nicht bekömmlich empfunden, verursachen ein nicht erwünschtes Aufstoßen und riechen und schmecken unangenehm nach Fisch. Kapseln werden auch aus Angst vor Prionen, einem vegetarischen oder veganen Lebensstil oder aus religiösen Gründen abgelehnt. Auch die direkte Einnahme öliger Substanzen im Allgemeinen sowie Omega-3-fettsäurehaltiger Öle im Speziellen wird häufig als unangenehm empfunden und/oder ist negativ behaftet, was zu einer schlechten Compliance bzw. weiterhin mangelnden Versorgung mit Omega-3-Fettsäuren führt. Dies gilt insbesondere auch für Personen, die sich der Wichtigkeit dieser Omega-3-Fettsäuren nicht bewusst sind. Letztlich gewährleistet ein zweiphasiges System mit Algenöl eine effiziente, positiv wahrgenommene und sichere Versorgung mit Omega-3-Fettsäuren und weist nicht die beschriebenen Probleme bekannter Zubereitungen auf.

In bestimmten bevorzugten Ausführungsformen enthält das zweiphasige System Sojaöl und/oder Sonnenblumenöl und/oder Borretschöl und/oder Leinöl und/oder Weizenkeimöl und/oder Sägepalmenöl und/oder Roggenextrakt.

Darin enthaltene Phytosterole regen das Haarwachstum an. In bestimmten Ausführungsformen enthält das zweiphasige System mindestens ein Phytosterol als isolierte Verbindung oder als Inhaltsstoff anderer Öle oder Fette. Ferner ist auch eine solche Zusammensetzung reich an Omega-3-Fettsäuren. Sie ist aber bekömmlicher und verursacht weniger Aufstoßen als eine Zusammensetzung mit Fischöl. Die Zusammensetzung weist vor allem nicht den typischen Geruch und Geschmack von Fisch auf. Darüber hinaus ist sie für eine vegetarische oder vegane Ernährung geeignet. Omega-3-Fettsäuren wie beispielsweise die Docosahexaensäure (DHA) und die Eicosapentaensäure (EPA) bilden einen wichtigen Teil der menschlichen Ernährung. Häufig wird der Bedarf an Omega-3-Fettsäuren nicht durch die Ernährungsgewohnheiten bzw. -möglichkeiten erfüllt. Erhältliche Omega-3-Präparate, insbesondere in Form von Kapseln mit Fischöl, werden häufig als nicht bekömmlich empfunden, verursachen ein nicht erwünschtes Aufstoßen und riechen und schmecken unangenehm nach Fisch. Kapseln werden auch aus Angst vor Prionen, einem vegetarischen oder veganen Lebensstil oder aus religiösen Gründen abgelehnt. Auch die direkte Einnahme öliger Substanzen im Allgemeinen sowie Omega-3-fettsäurehaltiger Öle im Speziellen wird häufig als unangenehm empfunden und/oder ist negativ behaftet, was zu einer schlechten Compliance bzw. weiterhin mangelnden Versorgung mit Omega-3-Fettsäuren führt. Dies gilt insbesondere auch für Personen, die sich der Wichtigkeit dieser Omega-3-Fettsäuren nicht bewusst sind.

In einer bestimmten bevorzugten Ausführungsform enthält das zweiphasige System Ubichinon-10 und/oder Ubichinol.

Ubichinon-10 und Ubichinol ist eine lipophile Verbindung und essentiell für die humane Atmungskette. Die reduzierte Form Ubichinol kann als Radikalfänger dienen. Das zweiphasige System kann als Trägersystem auch eine Versorgung mit Ubichinon-10 und Ubichinol sicherstellen.

In einer bestimmten bevorzugten Ausführungsform enthält das zweiphasige System Docosahexaensäure und Eicosapentaensäure.

Das zweiphasige System stellt eine effiziente Versorgung des Körpers mit Omega-3-Fettsäuren bereit. Die spezielle Zusammensetzung und Herstellung des zweiphasigen Systems bedingt, dass die oxidations- und hydrolyseempfindlichen Omega-3-Fettsäuren trotz Anwesenheit von Wasser und Luft überraschenderweise stabil vorliegen.

Das zweiphasige System enthält keine Inhaltsstoffe tierischer Herkunft. Mit anderen Worten eignet sich das zweiphasige System für einen vegetarischen und/oder veganen Lebensstil, insbesondere eine vegetarische und/oder vegane Ernährung. Vegetarier und vor allem Veganer leiden aufgrund ihres eingeschränkten Nahrungsmittelsangebotes oft an einem Mangel an bestimmten Verbindungen und entsprechenden Mangelerscheinungen. Vorteilhaft ist, dass diesen Menschen ein weiteres Produkt angeboten werden kann, um eine ausgewogenere Ernährung sicherzustellen.

Vorzugsweise bildet die wässrige Phase die äußere Phase.

In manchen Ausführungsformen besteht die wässrige Phase aus Wasser und Glycerin.

In einer bestimmten Ausführungsform enthält die wässrige Phase Monosaccharide, Disaccharide, Maltit, Maltodextrin, Threit, Pentite und/oder Hexite. Auch diese Verbindungen können die Wasseraktivität in der wässrigen Phase senken und zur Konservierung beitragen.

In einer bestimmten Ausführungsform enthält das zweiphasige System Orangenöl.

Somit wird der Geschmack und Geruch des zweiphasigen Systems verbessert und der Geschmack und Geruch unangenehm wahrgenommener Inhaltsstoffe kaschiert. Dadurch wird die Compliance gesteigert und die Annahme durch den Anwender verbessert.

In bestimmten Ausführungsformen liegt die lipophile Phase, bevorzugt zu mindestens 95 Gew.-%, in mizellierter Form vor.

Die lipophile Phase bildet Mizellen mit einer mittleren Tröpfchengröße zwischen 10 nm und 250 nm, wobei die mittlere Tröpfchengröße bevorzugt durch Photonenkorrelationsspektroskopie (PCS) bestimmt wird.

Dadurch kann ein besonders transparentes zweiphasiges System erhalten werden. Transparente zweiphasige Systeme werden von Anwendern bevorzugt.

Bevorzugt liegt die lipophile Phase nicht in Liposomen vor.

Vorzugsweise enthält das zweiphasige System keine weiteren konservierenden Verbindungen, insbesondere keine ein- oder zweiwertigen Alkohole, p-Hydroxybenzoesäureester, und/oder keine weiteren Tenside, insbesondere keine ethoxylierten und/oder synthetischen Tenside.

Dadurch ist das zweiphasige System besonders mit Blick auf Kinder, Allergiker, Alkoholiker und Schwangere gut verträglich und wird besonders positiv angenommen. Insbesondere in der Schwangerschaft wird die Gabe von Omega-3-Fettsäuren empfohlen. Gleichzeitig wird gerade in der Schwangerschaft angestrebt, auf schädliche und/oder nicht natürlich vorkommende Verbindungen zu verzichten. Daher bietet das zweiphasige System eine innovative Lösung.

Die Erfindung beruht ferner auf dem Gedanken, ein Verfahren zur Herstellung eines erfindungsgemäßen zweiphasigen Systems anzugeben. Das Verfahren umfasst die Schritte:
a) Rühren einer Zusammensetzung, enthaltend Lecithine und eine wässrige Lösung von Glycerin, wobei die wässrige Lösung 75 Gew.-% bis 99 Gew.-% Glycerin enthält,
b) Zugeben von Lipid, um ein Gemisch zu erhalten, derart, dass in dem Gemisch
   - 0,02 Gew.-% bis 7,77 Gew.-%, besonders bevorzugt 0,02 Gew.-% bis 7,52 Gew.-%, Lipid enthalten sind;
   - die Summe an Gew.-% von Lecithinen und Lipid an dem Gemisch 0,5 Gew.-% bis 8,25 Gew.-%, besonders bevorzugt 0,5 Gew.-% bis 8,0 Gew.-%, beträgt
   - 68 Gew.-% bis 91 Gew.-% Glycerin enthalten sind,
   wobei das Zugeben von Lipid das Zugeben mindestens eines von Algenöl, Sojaöl, Sonnenblumenöl, Borretschöl, Leinöl, Weizenkeimöl, Sägepalmenöl, Roggenextrakt Ubichinon-10 und Ubiquinol umfasst,
c) Rühren des Gemisches und Homogenisieren unter Hochdruck, um das zweiphasige System zu erhalten, wobei die lipophile Phase Mizellen mit einer mittleren Tröpfchengröße zwischen 10 nm und 250 nm bildet.

Bevorzugt ist ein Algenöl, insbesondere ein Algenöl aus Ulkenia sp. und/oder Schizochytrium sp., und/oder Ubichinon-10 und/oder Ubiquinol und/oder Sojaöl und/oder Sonnenblumenöl und/oder Borretschöl und/oder Leinöl und/oder Weizenkeimöl und/oder Sägepalmenöl und/oder Roggenextrakt.

Bevorzugt erfolgt das Zugeben von Lipid derart, dass das Gemisch 0,48 Gew.-% bis 8,46 Gew.-%, bevorzugt 0,48 Gew.-% bis 8,21 Gew.-%, besonders bevorzugt 0,48 Gew.-% bis 7,96 Gew.-%, Lecithine enthält.

Bevorzugt erfolgt das Zugeben von Lipid derart, dass in dem Gemisch das Massenverhältnis an Lecithinen zu Lipid zwischen 8:1 und 1:20, besonders bevorzugt zwischen 1:1 und 1:12, beträgt.

In bestimmten Ausführungsformen besteht die wässrige Lösung von Glycerin aus Wasser und Glycerin.

Das Zugeben von Lipid umfasst in bestimmten Ausführungsformen das Zugeben von Docosahexaensäure und Eicosapentaensäure, insbesondere als Bestandteil eines Öles.

Das Zugeben von Lipid umfasst in bestimmten Ausführungsformen das Zugeben von Orangenöl.

In bestimmten Ausführungsformen enthält die wässrige Lösung Monosaccharide, Disaccharide, Maltit, Maltodextrin, Threit, Pentite und/oder Hexite.

Bevorzugt werden keine weiteren konservierenden Verbindungen, insbesondere keine ein- oder zweiwertigen Alkohole, p-Hydroxybenzoesäureester, und/oder keine weiteren Tenside, insbesondere keine ethoxylierten und/oder synthetischen Tenside, zugegeben.

Die Verfahrenstemperatur wird bevorzugt den Lecithinen und dem zu solubilisierenden Lipid angepasst. Bei hydrierten Lecithinen sind Verarbeitungstemperaturen zwischen 40 °C und 80 °C bevorzugt, bei ungesättigten Lecithinen kann bei Raumtemperatur gearbeitet werden. Bevorzugt wird oberhalb des Schmelzpunktes der Lipide gearbeitet.

Bevorzugt erfolgt das Homogenisieren mit einem Hochdruckhomogenisator, Ultraschall und/oder einem Rotor-Stator-Mischer, insbesondere mit einem Hochdruckhomogenisator.

Ein Hochdruckhomogenisator ergibt transparentere zweiphasige Systeme, da die innere Phase feiner dispergiert wird. Mit einem Hochdruckhomogenisator wird erreicht, dass die lipophile Phase, bevorzugt zu mindestens 95 Gew.-%, in mizellierter Form vorliegt und/oder dass die lipophile Phase Mizellen mit einer mittleren Tröpfchengröße zwischen 5 nm und 500 nm, bevorzugt 10 nm und 250 nm, bildet, wobei die mittlere Tröpfchengröße bevorzugt per Photonenkorrelationsspektroskopie (PCS) bestimmt wird. Überraschenderweise ist der Grad an Autoxidation und Hydrolyse der Öle, Fette bzw. Lipide gering, obwohl mit einem Hochdruckhomogenisator Luft, Sauerstoff und Wasser eingearbeitet wird.

Bevorzugt wird das Verfahren, insbesondere das Rühren des Gemisches und das Homogenisieren unter Hochdruck (Schritt c), unter Inertgas durchgeführt.

Zu weiteren Ausführungsformen, Vorteilen und Erläuterungen wird auf die Ausführungen zu dem zweiphasigen System verwiesen.

Die Erfindung beruht ferner auf dem Gedanken, die Verwendung eines erfindungsgemäßen zweiphasigen Systems als Nahrungsergänzungsmittel oder kosmetisches Präparat anzugeben. Besonders bevorzugt ist die Verwendung als Nahrungsergänzungsmittel.

Das zweiphasige System ist zur Verwendung als Nahrungsergänzungsmittel und/oder kosmetisches Präparat besonders geeignet, da sie die Nachteile üblicher Produkte überwindet. Nur mittels natürlich vorkommender Inhaltsstoffe und ohne die Verwendung ein- oder zweiwertiger Alkohole, synthetischer und/oder ethoxylierter Tenside oder Konservierungsmittel wird ein physikalisch, chemisch und mikrobiell stabiles zweiphasiges System gebildet. Dadurch wird das zweiphasige System physiologisch und psychologisch besonders positiv angenommen und ist besonders gut verträglich. Positiv ist auch, dass das zweiphasige System transparent ist und daher eine ansprechende Optik aufweist. Der Geschmack und Geruch wird nicht als unangenehm, sondern als sehr gut empfunden. Bei der oralen Anwendung wird ein Aufstoßen verhindert. Da keine (Gelatine-)Kapseln verwendet werden, stehen die erfindungsgemäßen Präparate auch Personen zur Verfügung, die Kapseln aus Angst vor Prionen, wegen eines vegetarischen oder veganen Lebensstils oder aus religiösen Gründen ablehnen. Das zweiphasige System ist tropffähig und weist eine vorteilhafte Viskosität auf. Ferner erlaubt es das zweiphasige System, gleichzeitig hydrophile und hydrophobe Verbindungen aufzunehmen, um die Eigenschaften des zweiphasigen Systems weiter zu modifizieren oder um als Trägersystem für bestimmte Verbindungen zu dienen.

Die Verwendung umfasst bevorzugt die topische oder orale, besonders bevorzugt die orale, Anwendung.

Zu weiteren Ausführungsformen, Vorteilen und Erläuterungen wird auf die Ausführungen zu dem zweiphasigen System und dessen Herstellungsverfahren verwiesen.

Die Erfindung wird nachfolgend anhand von Ausführungsbeispielen näher erläutert.

### Beispiel 1:

0,6 g einer Lecithinfraktion aus Sonnenblumen mit einem Gehalt an Phosphatidylcholin von 50 Gew.-% wird in 94,4 g einer 80 %-igen (Gew.-%), wässrigen Glycerinlösung unter Rühren vollständig dispergiert. Dieser Dispersion werden nun 5,0 g Ubichinon-10 zugegeben und bei 50 °C zur Homogenität gerührt. Abschließend erfolgt eine Homogenisation mittels Hochdruckhomogenisator. Das Ergebnis ist eine transparente, niedrigviskose, mizellare Dispersion mit 5,0 Gew.-% Ubichinon-10, wobei die Summe an Gew.-% Lecithinen und Ubichinon-10 an der mizellaren Dispersion 5,6 Gew.-% beträgt. Ferner enthält die mizellare Dispersion 75,5 Gew.-% Glycerin.

Das erhaltene zweiphasige System eignet sich insbesondere zur topischen Anwendung.

### Beispiel 2:

1,2 g einer Lecithinfraktion aus Soja mit einem Gehalt von Phosphatidylcholin von 80 Gew.-% wird in 92,3 g einer 75 %-igen (Gew.-%), wässrigen Glycerinlösung unter Rühren vollständig dispergiert. 6,5 g einer 70 %-igen alpha-Linolensäurelösung in Triglyceriden werden unter Rühren zugegeben und homogen verteilt. Diese Vormischung wird abschließend mit Hilfe eines Hochdruckhomogenisators homogenisiert. Das Resultat ist ein niedrigviskoses, transparentes, mizellares Solubilisat mit 4,55 Gew.-% alpha-Linolensäure, wobei die Summe an Gew.-% Lecithinen und alpha-Linolensäure an der mizellaren Dispersion 5,75 Gew.-% beträgt. Ferner enthält die mizellare Dispersion 69,2 Gew.-% Glycerin.

In dem erhaltenen zweiphasigen System liegt die lipophile Phase zu mindestens 95 Gew.-%, in mizellierter Form vor und bildet Mizellen mit einer mittleren Tröpfchengröße zwischen 10 nm und 250 nm.

### Beispiel 3:

1,2 g einer Lecithinfraktion aus Soja mit einem Gehalt von Phosphatidylcholin von 80 Gew.-% wird in 100 g einer 75 %-igen (Gew.-%), wässrigen Glycerinlösung unter Rühren vollständig dispergiert. 6,5 g eines Algenöles aus Ulkenia sp. und Schizochytrium sp. werden unter Rühren zugegeben und homogen verteilt. Diese Vormischung wird abschließend mit Hilfe eines Hochdruckhomogenisators homogenisiert. Das Resultat ist ein niedrigviskoses, transparentes, mizellares Solubilisat mit 6,0 Gew.-% Algenöl aus Ulkenia sp. und Schizochytrium sp., wobei die Summe an Gew.-% Lecithinen und Algenöl aus Ulkenia sp. und Schizochytrium sp. an der mizellaren Dispersion 7,1 Gew.-% beträgt. Ferner enthält die mizellare Dispersion 70 Gew.-% Glycerin.

Das erhaltene zweiphasige System enthält Docosahexaensäure und Eicosapentaensäure und eignet sich insbesondere zur oralen Anwendung bei einem veganen Lebensstil.

## Patentansprüche

1. Zweiphasiges System, umfassend eine lipophile Phase und eine wässrige Phase, wobei
- das zweiphasige System Lecithine enthält;
- das zweiphasige System 0,02 Gew.-% bis 7,77 Gew.-%, besonders bevorzugt 0,02 Gew.-% bis 7,52 Gew.-%, Lipid enthält;
- die Summe an Gew.-% von Lecithinen und Lipid an dem zweiphasigen System 0,5 Gew.-% bis 8,25 Gew.-%, besonders bevorzugt 0,5 Gew.-% bis 8,0 Gew.-%, beträgt;
- das zweiphasige System 68 Gew.-% bis 91 Gew.-% Glycerin enthält,
wobei das zweiphasige System keine Inhaltsstoffe tierischer Herkunft enthält,
wobei die lipophile Phase Mizellen mit einer mittleren Tröpfchengröße zwischen 10 nm und 250 nm bildet,
wobei die wässrige Phase 75 Gew.-% - 99 Gew.-% Glycerin enthält,
wobei das zweiphasige System einen Bestandteil ausgewählt aus Algenöl, Sojaöl, Sonnenblumenöl, Borretschöl, Leinöl, Weizenkeimöl, Sägepalmenöl, Roggenextrakt, Ubichinon-10 und Ubiquinol enthält.

2. Zweiphasiges System nach Anspruch 1, wobei das zweiphasige System 0,48 Gew.-% bis 8,21 Gew.-%, besonders bevorzugt 0,48 Gew.-% bis 7,96 Gew.-%, Lecithine enthält.

3. Zweiphasiges System nach einem der vorherigen Ansprüche, wobei das zweiphasige System Docosahexaensäure und Eicosapentaensäure enthält.

4. Zweiphasiges System nach einem der vorherigen Ansprüche, wobei das zweiphasige System für einen veganen Lebensstil geeignet ist.

5. Zweiphasiges System nach einem der vorherigen Ansprüche, wobei die wässrige Phase die äußere Phase bildet.

6. Zweiphasiges System nach einem der vorherigen Ansprüche, wobei die wässrige Phase aus Wasser und Glycerin besteht.

7. Zweiphasiges System nach einem der vorherigen Ansprüche, wobei das zweiphasige System Orangenöl enthält.

8. Zweiphasiges System nach einem der vorherigen Ansprüche, wobei die lipophile Phase, bevorzugt zu mindestens 95 Gew.-%, in mizellierter Form vorliegt.

9. Zweiphasiges System nach einem der vorherigen Ansprüche, wobei das zweiphasige System keine weiteren konservierenden Verbindungen enthält, insbesondere keine ein- oder zweiwertigen Alkohole, p-Hydroxybenzoesäureester, und/oder keine weiteren Tenside, insbesondere keine ethoxylierten und/oder synthetischen Tenside.

10. Verfahren zur Herstellung des zweiphasigen Systems nach einem der Ansprüche 1-9, umfassend die Schritte:
a) Rühren einer Zusammensetzung, enthaltend Lecithine und eine wässrige Lösung von Glycerin, wobei die wässrige Lösung 75 Gew.-% bis 99 Gew.-% Glycerin enthält,
b) Zugeben von Lipid, um ein Gemisch zu erhalten, derart, dass in dem Gemisch
- 0,02 Gew.-% bis 7,77 Gew.-%, besonders bevorzugt 0,02 Gew.-% bis 7,52 Gew.-%, Lipid enthalten sind;
- die Summe an Gew.-% von Lecithinen und Lipid an dem Gemisch 0,5 Gew.-% bis 8,25 Gew.-%, besonders bevorzugt 0,5 Gew.-% bis 8,0 Gew.-%, beträgt;
- 68 Gew.-% bis 91 Gew.-% Glycerin enthalten sind,
wobei das Zugeben von Lipid das Zugeben mindestens eines von Algenöl, Sojaöl, Sonnenblumenöl, Borretschöl, Leinöl, Weizenkeimöl, Sägepalmenöl, Roggenextrakt Ubichinon-10 und Ubiquinol umfasst;
c) Rühren des Gemisches und Homogenisieren unter Hochdruck, um das zweiphasige System zu erhalten, wobei die lipophile Phase Mizellen mit einer mittleren Tröpfchengröße zwischen 10 nm und 250 nm bildet.

11. Zweiphasiges System nach einem der Ansprüche 1-9 zur Verwendung als Nahrungsergänzungsmittel oder kosmetisches Präparat.

## Claims

1. Two-phase system, comprising a lipophilic phase and an aqueous phase, wherein
- the two-phase system contains lecithins;
- the two-phase system contains 0.02 wt.% to 7.77 wt.%, particularly preferably 0.02 wt.% to 7.52 wt.%, of lipid;
- the total wt.% of lecithins and lipid in the two-phase system is from 0.5 wt.% to 8.25 wt.%, particularly preferably from 0.5 wt.% to 8.0 wt.%;
- the two-phase system contains 68 wt.% to 91 wt.% of glycerol,
wherein the two-phase system does not contain ingredients of animal origin,
wherein the lipophilic phase forms micelles with an average droplet size between 10 nm and 250 nm,
wherein the aqueous phase contains 75 wt.% to 99 wt.% glycerol,
wherein the two-phase system contains an ingredient selected from algae oil, soybean oil, sunflower oil, borage oil, linseed oil, wheat germ oil, saw palmetto oil, rye extract, ubiquinone-10 and ubiquinol.

2. Two-phase system according to claim 1, wherein the two-phase system contains 0.48 wt.% to 8.21 wt.%, particularly preferably 0.48 wt.% to 7.96 wt.%, of lecithins.

3. Two-phase system according to one of the preceding claims, wherein the two-phase system comprises docosahexaenoic acid and eicosapentaenoic acid.

4. Two-phase system according to one of the preceding claims, wherein the two-phase system is suitable for a vegan lifestyle.

5. Two-phase system according to one of the preceding claims, wherein the aqueous phase forms the outer phase.

6. Two-phase system according to one of the preceding claims, wherein the aqueous phase consists of water and glycerol.

7. Two-phase system according to one of the preceding claims, wherein the two-phase system comprises orange oil.

8. Two-phase system according to one of the preceding claims, wherein the lipophilic phase, preferably at least 95 wt.%, is in micellated form.

9. Two-phase system according to one of the preceding claims, wherein the two-phase system contains no further preservative compounds, in particular no monohydric or dihydric alcohols, p-hydroxybenzoic acid esters, and/or no further surfactants, in particular no ethoxylated and/or synthetic surfactants.

10. Method for producing the two-phase system according to one of claims 1-9, comprising the steps of:
a) stirring a composition, containing lecithins and an aqueous solution of glycerol, wherein the aqueous solution contains 75 wt.% to 99 wt.% of glycerol,
b) adding lipid to obtain a mixture, such that in the mixture
- 0.02 wt.% to 7.77 wt.%, particularly preferably 0.02 wt.% to 7.52 wt.%, of lipid are present;
- the total wt.% of lecithins and lipid in the mixture is from 0.5 wt.% to 8.25 wt.%, particularly preferably from 0.5 wt.% to 8.0 wt.%;
- 68 wt.% to 91 wt.% of glycerol are present,
wherein the adding of lipid comprises adding at least one of algae oil, soybean oil, sunflower oil, borage oil, linseed oil, wheat germ oil, saw palmetto oil, rye extract, ubiquinone-10 and ubiquinol;
c) stirring the mixture and homogenization under high pressure to obtain the two-phase system, wherein the lipophilic phase forms micelles with an average droplet size between 10 nm and 250 nm.

11. The two-phase system according to one of claims 1-9 for use as a dietary supplement or cosmetic preparation.

## Revendications

1. Système à deux phases comprenant une phase lipophile et une phase aqueuse, dans lequel
- le système à deux phases contient des lécithines ;
- le système à deux phases contient entre 0,02 % en poids et 7,77 % en poids, de préférence, en particulier, entre 0,02 % en poids et 7,52 % en poids, de lipide ;
- le somme des pourcentages en poids des lécithines et du lipide dans le système à deux phases représente entre 0,5 % en poids et 8,25 % en poids, de préférence, en particulier, entre 0,5 % en poids et 8,0 % en poids ;
- le système à deux phases contient entre 68 % en poids et 91 % en poids de glycérine,
le système à deux phases ne contenant aucun ingrédient d'origine animale,
la phase lipophile formant des micelles ayant une taille de gouttelettes moyenne comprise entre 10 nm et 250 nm,
la phase aqueuse contenant entre 75 % en poids et 99 % en poids de glycérine,
le système à deux phases contenant un ingrédient sélectionné parmi l'huile d'algues, l'huile de soja, l'huile de tournesol, l'huile de bourrache, l'huile de lin, l'huile de germe de blé, l'huile de palmier de Floride, un extrait de seigle, l'ubiquinone 10 et l'ubiquinol.

2. Système à deux phases selon la revendication 1, lequel système à deux phases contient entre 0,48 % en poids et 8,21 % en poids, de préférence, en particulier, entre 0,48 % en poids et 7,96 % en poids, de lécithines.

3. Système à deux phases selon l'une des revendications précédentes, lequel système à deux phases contient de l'acide docosahexaénoïque et de l'acide eicosapentaénoïque.

4. Système à deux phases selon l'une des revendications précédentes, lequel système à deux phases convient pour un mode de vie végétalien.

5. Système à deux phases selon l'une des revendications précédentes, dans lequel la phase aqueuse forme la phase externe.

6. Système à deux phases selon l'une des revendications précédentes, dans lequel la phase aqueuse se compose d'eau et de glycérine.

7. Système à deux phases selon l'une des revendications précédentes, lequel système à deux phases contient de l'huile essentielle d'orange.

8. Système à deux phases selon l'une des revendications précédentes, dans lequel la phase lipophile se présente sous forme micellaire, de préférence à au moins 95 % du poids.

9. Système à deux phases selon l'une des revendications précédentes, lequel système à deux phases ne contient pas d'autres composés conservateurs, en particulier pas d'alcools mono- ou divalents, d'esters d'acide p-hydroxybenzoïqueni aucun autre agent tensioactif, en particulier pas de tensioactifs éthoxylés et/ou de synthèse.

10. Procédé pour la fabrication du système à deux phases selon l'une des revendications 1 à 9, comprenant les étapes suivantes :
a) agitation d'une composition contenant des lécithines et une solution aqueuse de glycérine, laquelle solution aqueuse contient entre 75 % en poids et 99 % en poids de glycérine ;
b) ajout de lipide pour obtenir un mélange tel que
- le mélange contient entre 0,02 % en poids et 7,77 % en poids, de préférence, en particulier, entre 0,02 % en poids et 7,52 % en poids, de lipide ;
- la somme des pourcentages en poids des lécithines et du lipide dans le mélange représente entre 0,5 % en poids et 8,25 % en poids, de préférence, en particulier, entre 0,5 % en poids et 8,0 % en poids ;
- le mélange contient entre 68 % en poids et 91 % en poids de glycérine,
l'ajout de lipide comprenant l'ajout d'au moins une des substances suivantes : huile d'algues, huile de soja, huile de tournesol, huile de bourrache, huile de lin, huile de germe de blé, huile de palmier de Floride, extrait de seigle, ubiquinone 10 et ubiquinol ;
c) agitation du mélange et homogénéisation sous haute pression afin d'obtenir le système à deux phases, la phase lipophile formant des micelles dont la taille de gouttelettes moyenne est comprise entre 10 nm et 250 nm.

11. Système à deux phases selon l'une des revendications 1 à 9, destiné à être utilisé comme complément alimentaire ou comme préparation cosmétique.
